(19) Europäisches Patentamt

European Patent Office

Office européen des brevets

(11) **EP 1 146 862 B1**

(12) **EUROPÄISCHE PATENTSCHRIFT**

(45) Veröffentlichungstag und Bekanntmachung des Hinweises auf die Patenterteilung:
**23.04.2003   Patentblatt 2003/17**

(51) Int Cl.[7]: **A61K 9/16**, A61K 9/50, A61K 9/00, A61K 9/20, A61K 9/48

(21) Anmeldenummer: **99900623.2**

(22) Anmeldetag: **29.01.1999**

(86) Internationale Anmeldenummer:
**PCT/IB99/00180**

(87) Internationale Veröffentlichungsnummer:
**WO 00/044353 (03.08.2000 Gazette 2000/31)**

(54) **PHARMAZEUTISCHE ZUSAMMENSETZUNGEN**

PHARMACEUTICAL COMPOSITIONS

COMPOSITIONS PHARMACEUTIQUES

(84) Benannte Vertragsstaaten:
**AT BE CH DE DK ES FI FR GB GR IE IT LI LU NL PT SE**

(43) Veröffentlichungstag der Anmeldung:
**24.10.2001   Patentblatt 2001/43**

(73) Patentinhaber: **Disphar International B.V.**
**7255 PZ Hengelo (Gld.) (NL)**

(72) Erfinder:
• **HUBER, Gerald**
**D-74613 Öhringen (DE)**

• **GRUBER, Peter**
**D-79104 Freiburg (DE)**

(74) Vertreter: **Zimmermann, Hans, Dr. et al**
**A. Braun Braun Héritier Eschmann AG**
**Holbeinstrasse 36-38**
**4051 Basel (CH)**

(56) Entgegenhaltungen:
| | |
|---|---|
| **EP-A- 0 220 143** | **EP-A- 0 365 947** |
| **WO-A-91/18590** | **WO-A-91/19483** |
| **WO-A-97/25980** | **WO-A-98/20858** |
| **US-A- 5 178 868** | **US-A- 5 607 695** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft verbesserte Retardformulierungen zur Freisetzung von pharmazeutischen Wirkstoffen, wie beispielsweise 5-Aminosalicylsäure, im Magen-Darm-Trakt, vorzugsweise im Darmtrakt oder Colon, sowie ein Verfahren zur Herstellung solcher Formulierungen.

Oral verabreichbare Formulierungen von 5-Aminosalicylsäure zur Behandlung von Colitis ulcerosa und Morbus Crohn sind bereits aus WO-A-81/02671 bekannt. Die dort offenbarte Tablettenformulierung wurde erhalten durch Granulieren von 250 g 5-Aminosalicylsäure mit einer Lösung von 25 g Polyvinylpyrrolidon in Isopropanol, anschliessendes Überziehen des getrockneten Granulates mit 45 g Ethylcellulose, Mischen des überzogenen Granulates mit 3 g Natriumstearat, 27 g Talkum und 300 g eines Granulates aus mikrokristalliner Cellulose, Kartoffelstärke und Polyvinylpyrrolidon und Verpressen der Mischung zu Tabletten mit einem Tablettengewicht von 650 mg und einem Wirkstoffgehalt von 250 mg. Solche Tabletten sind unter dem Namen Pentasa® (Ferring, Dänemark) im Handel erhältlich. Nachteilig an dieser Formulierung ist jedoch der relativ hohe Anteil an Hilfsstoffen von über 60 Gew.-% und der niedrige Wirkstoffgehalt im Vergleich zu den heute üblichen Tages-Dosen von etwa 1,5-4,5 g. Ferner werden Granulatteilchen bei der Tablettierung leicht zerstört und verändern dadurch das Freigabeverhalten des Wirkstoffes.

Daneben sind eine Reihe von Vorschlägen bekannt geworden, mit denen versucht wurde, eine gezieltere oder kontrolliertere Freisetzung von Wirkstoffen im Darmtrakt oder andere Vorteile zu erreichen.

Beispielsweise offenbart WO-A-83/00435 oral verabreichbare Zusammensetzungen, die mit einem unterhalb pH 7 unlöslichen, aber im Colon löslichen anionischen Polymer überzogen sind, wobei Kapseln oder Tabletten beschrieben werden, die 5-Aminosalicylsäure, Prednisolon oder Indomethacin enthalten und mit einem Überzug, enthaltend Eudragit S100, versehen sind. Die offenbarten Arzneiformen sind überzogene Kapseln oder überzogene Tabletten, d.h. monolithische Arzneiformen. Die Freisetzung soll selektiv im Colon erfolgen, wofür bislang nur sehr teuer herzustellende Coatingmembranen mit einer Lackschichtdicke von 60-150 μm erforderlich sind.

Die Möglichkeit 5-Aminosalicylsäure-Formulierungen magensaftresistent zu umhüllen, wird ebenfalls in WO-A-92/16206 und DE-A-31 51 196 erwähnt. Letztere Offenbarung betrifft gut lösliche Arzneimittelzubereitungen, die durch Vermischen von 5-Aminosalicylsäure mit basischen Hilfsstoffen und/oder Puffergemischen erhalten werden. Demgegenüber wird in WO-A-94/28911 eine Zusammensetzung, die ein pH-regulierendes, im wesentlichen unlösliches, alkalisches Material enthält und gewünschtenfalls einen enterischen Überzug aufweisen kann, vorgeschlagen und als Beispiel eine Tablettenformulierung angegeben, die aus einem mit Eudragit L12,5P überzogenen Granulat von Calciumcarbonat durch Mischen und Tablettieren zusammen mit einem mit Ethylcellulose überzogenen Granulat von 5-Aminosalicylsäure erhalten wurde.

Aus EP-A-0 671 168 ist eine orale Zusammensetzung zur kontrollierten Freisetzung im Darmtrakt bekannt, bei der eine Manteltablette mit einem wirkstoffhaltigen Kern hergestellt wird. Die Umhüllung enthält Polymerpulver, welches zu einer Magensaftresistenz führt. Die Herstellung von Manteltabletten ist jedoch teuer und benötigt spezielle Tablettenpressen. Eine ähnliche Methode zur Herstellung einer monolithischen, magensaftresisten Arzneiform ist auch in EP-A-0 671 167 beschrieben, wobei aber für die Umhüllung ein pH-unabhängig wasserlösliches Polymer verwendet und dann die umhüllte Tablette noch mit einem enterischen Polymerfilm überzogen wird.

Ferner wurde auch bereits die Kombination von enterischen und unlöslichen Materialien in einer Überzugsschicht vorgeschlagen. Beispielsweise beschreibt EP-A-0 040 590 oral verabreichbare Zusammensetzungen, umfassend einen wirkstoffhaltigen Kern und einen Überzug, wobei letzterer ein nur über pH 5,5 lösliches anionisches Acrylpolymer und ein wasserunlösliches quaternäres, ammonium-substituiertes Acrylpolymer im Gewichtsverhältnis von 10-85:90-15 und ferner vorzugsweise einen Fettalkohol oder eine Fettsäure als Weichmacher enthält. WO-A-92/14452 offenbart zwar eine Kapselformulierung zur selektiven Wirkstofffreisetzung im Darm, in der sowohl das in der Kapsel vorhandene Granulat als auch die Kapsel selbst mit einem im Darmsaft löslichen Material überzogen sind; wie erwähnt wird, kann aber gewünschtenfalls der Überzug des Granulates ein enterisches Material im Gemisch mit einem neutralen, unlöslichen, aber durchlässigen Polymer enthalten. Die Herstellung dieser Arzneiform ist teuer, und sie führt zu einer "single unit dosage form", deren Verweilzeit im Magen hohen zeitlichen Schwankungen unterliegen kann.

Demgegenüber wird in GB-A-2 134 785 eine langsam freisetzende Formulierung von Pinacidil offenbart, die zwei Arten von Pellets umfasst, wobei die erste Pelletart mit einem im Gastrointestinaltrakt unlöslichen, aber durchlässigen Material und die zweite Pelletart mit einem bei niedrigem pH schwer löslichen, aber bei pH-Werten über 5-7,5 löslichen Material umhüllt ist. Die Pellets werden durch Aufsprühen einer Wirkstoffsuspension auf "Non Pareils" (Neutralpellets) hergestellt und wären für eine Verpressung zu einer Tablettenform nicht geeignet.

In WO-A-92/09270 wird ein Verfahren vorgeschlagen, welches die direkte Verwendung eines Extrudates in der Herstellung von Dosierungsformen ermöglichen soll und in welchem eine feuchte Masse von Wirkstoff und Hilfsstoffen extrudiert und das Extrudat mit einem wasserunlöslichen Material überzogen wird. Das Extrudat muss hierzu relativ viel Hilfsstoff enthalten und wäre zur Verpressung zu Tabletten ebenfalls mechanisch nicht ausreichend stabil.

WO-A-85/03437 beschreibt "Multiple Units"-Formulierungen mit kontrollierter Freisetzung, in denen wirkstoffhaltige Teilchen (Kristalle oder extrudierte Pellets) mit einem im wesentlichen wasserunlöslichen, aber wasserdiffundierbaren Überzug umhüllt sind, der aus einer oder zwei Schichten bestehen kann, wobei die innere bzw. einzige Schicht eine homogene Kombination eines wasserdisperigerbaren filmbildenden Mittels und einer polymeren, vorzugsweise wasserlöslichen Substanz aufweist, die dem Überzug plastische Deformierbarkeit verleihen (und damit signifikante Änderungen in der Freisetzungscharakteristik durch Verpressen zu Tabletten verhindern) soll, und die optionale äussere Schicht ein filmbildendes Mittel enthält, das eine Adhäsion zwischen den Teilchen bei erhöhter Temperatur verhindern und die Rieselfähigkeit verbessern soll. Die überzogenen Teilchen sind jedoch bei geringem Hilfsstoffanteil zur Verpressung zu Tabletten mechanisch nicht ausreichend stabil.

Weiterhin sind auch bereits verschiedene pharmazeutische Formulierungen vorgeschlagen worden, die sowohl einen enterischen als auch einen unlöslichen Überzug aufweisen. Beispielsweise werden in EP-A-0 148 811 Formulierungen von Wirkstoffen wie Chinidinsulfat beschrieben, die eine verbesserte Freisetzung unabhängig von der Löslichkeit des Wirkstoffes ermöglichen sollen und in denen ein Granulat aus Wirkstoff in Form einer schwachen Säure oder Base und Hilfsstoffen wie Lactose, Mannitol etc. mit einer Diffusionsmembran aus Ethylcellulose und/oder einem Copolymer von Polyethylmethacrylat-methylmethacrylat-trimethylammoniumethylmethacrylatchlorid und zusätzlich mit einer äusseren Schicht aus mindestens einem anionischen Polymer und/oder einer Fettsäure mit einem pKa von 4,5 bis 7 umhüllt ist. Die äussere Schicht soll gegen den Angriff durch Magensaft schützen, während die innere Membran eine langsame, aber kontrollierte Freisetzung ergeben soll, wodurch innert 7-10 Stunden 80-90% des Wirkstoffes in einer konstanten, pH-unabhängigen Weise freigesetzt werden sollen. Eine Formulierung mit einem wirkstoffhaltigen Kern, einem inneren, im Darmsaft schlecht löslichen Überzug aus Ethylcellulose, Hydroxypropylcellulose oder Carboxymethylcellulose und einem äusseren enterischen Überzug wird auch in EP-A-0 239 361 für Aspirin vorgeschlagen.

Demgegenüber werden in EP-A-0 212 745 Wirkstoffpartikel beschrieben, in denen der Kern, enthaltend ein Propionsäurederivat als Wirkstoff, mit einem inneren Überzug aus enterischem Acrylpolymer oder -copolymer und einem äusseren Überzug aus im Magen- und Darmsaft unlöslichem Methacrylsäurepolymer oder -copolymer umhüllt ist. Auf diese Weise soll die Abnahme der Überzugsdicke durch die Abnahme der Oberfläche der Partikel kompensiert und damit eine konstante Freisetzung erreicht werden.

Gemäss EP-A-0 453 001 soll ferner eine kontrollierte Freisetzung im Darm, insbesondere im Endteil des Ileums und Colons, dadurch erreicht werden, dass Teilchen eines entzündungshemmenden Mittels mit mindestens zwei Membranen umhüllt werden, wovon eine bei pH ≥ 5,5 löslich und die andere bei diesem pH unlöslich, aber durchlässig für Darmflüssigkeiten ist.

In WO-A-92/00732 wurde insofern ein anderer Weg gewählt, als zur Herstellung colon-selektiver Zusammensetzungen die Verwendung von Materialien wie Pektinen vorgeschlagen wurde, die selektiv durch im Colon normalerweise vorkommende Enzyme abbaubar sind. Die offenbarten Zusammensetzungen umfassen einen Matrix-Kern, in dem der Wirkstoff dispergiert ist, und einen Überzug, wobei sowohl der Matrix-Kern als auch der Überzug enzymatisch abbaubar sein sollen.

In WO-A-97/23199 wurde andererseits versucht, durch Wahl bestimmter Hilfsstoffe in Kombination einer optimaleren geometrischen Form von Granulatteilchen eine vorteilhafte Freisetzungscharakteristik für 5-Aminosalicylsäure zu erreichen und deren Bioverfügbarkeit sowohl im Dünndarm als auch im Dickdarm sicherzustellen. Die offenbarten Granulatteilchen weisen einen Kern, der 5-Aminosalicylsäure und ein sogenanntes Sphäronisierungsmittel, vorzugsweise mikrokristalline Cellulose, enthält, und einen Überzug aus einem semipermeablen Polymer, vorzugsweise Ethylcellulose, auf. Zudem sollen die Granulatteilchen im wesentlichen sphärisch sein und ein sogenanntes "Aspect ratio", das als Verhältnis der längsten zur kürzesten Abmessung der Teilchen definiert wird, von 1,00-1,25 aufweisen. In der Teilchenmatrix selbst ist kein magen- und darmsaftunlöslicher Lack eingearbeitet, und die beschriebenen Teilchen sind außerdem mechanisch nicht sehr stabil.

Die Herstellung sphärischer Teilchen ist auch bereits in WO-A-92/06679 beschrieben worden, wobei dort aber ein Schmelzgranulierverfahren vorgeschlagen wurde, in welchem eine Mischung, enthaltend Wirkstoff in kohäsiver Form und ein Bindemittel mit einem Schmelzpunkt zwischen 40°C und 100°C, unter Energiezufuhr mechanisch so verarbeitet wird, dass das Bindemittel schmilzt und die Mischung unter Bildung sphärischer Pellets granuliert.

Im Stand der Technik wurde somit vorwiegend versucht, durch Aufbringen eines magensaftresistenten Überzuges eine Freisetzung im Magen zu vermeiden oder durch Herstellung sphärischer Teilchen oder geeignete Kombination von Überzugsmaterialien die Retardierung zu verbessern. Letzteres erfordert jedoch zusätzliche Hilfsstoffe und/oder Verfahrensmassnahmen, während die Verwendung eines enterischen Überzugs nicht in jedem Fall eine selektive Wirkstofffreisetzung am gewünschten Ort im Magen-Darm-Trakt gewährleistet, da die pH-Werte im Magen-Darm-Trakt von Patient zu Patient zum Teil erheblich variieren können. Ausserdem kann die Verweilzeit (Residence time) von Tabletten im Magen und deren Durchgangszeit (Transit time) durch den Intestinaltrakt und das Colon bekanntlich grossen Schwankungen unterliegen, was eine gezielte Freisetzung ebenfalls erschwert.

Der Erfindung liegt daher die Aufgabe zugrunde, eine pharmazeutische Zusammensetzung zur langsamen Wirkstofffreisetzung im Magen-Darm-Trakt bereitzustellen, die die genannten Nachteile weitgehend vermeidet und die sich kostengünstig und mit hoher Reproduzierbarkeit herstellen lässt. Gemäss einer weiteren Aufgabe der vorliegenden Erfindung soll eine pharmazeutische Zusammensetzung zur Verfügung gestellt werden, die eine langsame Wirkstofffreisetzung im Darmtrakt auch bei hohem Wirkstoffgehalt und nur geringem Hilfsstoffanteil gestattet.

Diese Aufgabe wird erfindungsgemäss gelöst durch eine pharmazeutische Zusammensetzung zur langsamen Freisetzung von Wirkstoff im Magen-Darm-Trakt, umfassend mehrere überzogene wirkstoffhaltige Teilchen, die einen wirkstoffhaltigen Kern und einen Überzug, umfassend ein in Magen- und Darmsaft unlösliches Polymer, aufweisen, wobei der wirkstoffhaltige Kern der überzogenen Teilchen ein homogenes Gemisch, umfassend einen pharmazeutischen Wirkstoff und ein in Magen- und Darmsaft unlösliches Polymer, ist und einen mittleren inneren Porendurchmesser von höchstens 35 μm besitzt.

Die überzogenen wirkstoffhaltigen Teilchen der erfindungsgemässen Zusammensetzung weisen als Kern ein verdichtetes Gemisch, enthaltend pharmazeutischen Wirkstoff und magen- und darmsaftunlösliches Polymer, auf. Die Verdichtung äussert sich in einer Abnahme des mittleren inneren Porendurchmessers und des Porenvolumens bzw. der Porosität und lässt sich daher am besten durch den mittleren inneren Porendurchmesser und/oder die Porosität charakterisieren.

Der innere Porendurchmesser und die Porosität der wirkstoffhaltigen Kerne der erfindungsgemässen Zusammensetzung können mit einem Quantachrome oder Micromeritics Quecksilberporosimeter in einem Druckbereich von 1000 bis 4000 bar bestimmt werden. Die im Rahmen der vorliegenden Erfindung angegebenen Werte beziehen sich jeweils auf Messungen mit einem Quantachrome Poremaster (Firma Quantachrome, Odelzhausen, Deutschland) bei 1000 bis 4000 bar. Den mittleren Durchmesser der Poren erhält man hierbei aus dem Gleichgewichtsdruck, bei dem das Quecksilber in die Poren eindringt, wobei der Zusammenhang durch die Washburn-Gleichung beschrieben wird (vgl.: Dr. G. Huber, Dissertation 1993, Freie Universität Berlin, Fachbereich Pharmazie).

Durch die unten beschriebene erfindungsgemässe Kompaktierung des homogenen Gemisches, umfassend pharmazeutischen Wirkstoff und magen- und darmsaftunlösliches Polymer, wird dessen Porosität und der mittlere Durchmesser der inneren Poren signifikant verringert. Während die mittleren inneren Porendurchmesser bei üblichen Matrixgranulaten in der Regel bis zu etwa 100 μm betragen, weisen die erfindungsgemäss kompaktierten wirkstoffhaltigen Kerne einen mittleren inneren Porendurchmesser von zweckmässigerweise höchstens etwa 35 μm und vorzugsweise höchstens etwa 20 μm auf. Die Porosität wird bei der erfindungsgemässen Kompaktierung in der Regel um mindestens etwa 10% verringert. Die prozentuale Porosität ergibt sich aus der Rohdichte ρe (scheinbare Dichte, bestimmt mittels Quecksiberporosimetrie) und der wahren Dichte ρa (Feststoffdichte, bestimmt mittels Heliumpyknometrie) entsprechend der Beziehung: Porosität P = 100·(1-ρe/ρa). Die entsprechenden Werte liegen bei üblichen Matrixgranulaten typischerweise bei etwa 30%, während sie für die erfindungsgemäss kompaktierten wirkstoffhaltigen Kerne höchstens etwa 27%, beispielsweise etwa 10 bis 25%, betragen. Zudem steigt die Feststoffdichte der wirkstoffhaltigen Kerne durch die erfindungsgemässe Kompaktierung in der Regel um mindestens etwa 10% an.

Die erfindungsgemässe Zusammensetzung eignet sich vor allem zur gezielten Wirkstofffreisetzung im Darmtrakt und insbesondere im Colon. In einigen Fällen ist es jedoch erwünscht, dass die Wirkstofffreigabe bereits im Magen einsetzt, was mit der erfindungsgemässen Zusammensetzung ebenfalls erreicht werden kann. Beispielsweise ist es in Einzelfällen bei der Behandlung von hochsitzendem Morbus Crohn mit 5-Aminosalicylsäure erwünscht, dass im tieferen Teil des Magens Wirkstoff freigesetzt wird, um im kurzen Duodenaltrakt eine optimale Wirkung zu erzielen.

Die erfindungsgemässe Zusammensetzung hat den Vorteil, dass die Wirkstofffreisetzung weitestgehend in pH-unabhängiger Weise erfolgt und damit Einflüsse biologischer Unterschiede zwischen einzelnen Patienten fast völlig vermieden werden können. Zudem können die überzogenen wirkstoffhaltigen Teilchen als solche oder vorzugsweise in Tabletten oder anderen Dosierungsformen verabreicht werden, die im Magen rasch zerfallen und die überzogenen wirkstoffhaltigen Teilchen freigeben. Da die überzogenen wirkstoffhaltigen Teilchen eine Teilchengrösse (d.h. maximale Abmessung) von vorzugsweise etwa 0,1-3,0 mm, insbesondere etwa 0,2-2,5 mm und besonders bevorzugt etwa 0,3-2,0 mm aufweisen, ist in jedem Fall gewährleistet, dass sie den Magen sehr schnell durch den Pylorus verlassen. Die bei retardierten Tabletten naturbedingt vorkommenden grossen Schwankungen hinsichtlich der Verweilzeit im Magen und der Durchgangszeit durch den Intestinaltrakt und das Colon werden daher mit der erfindungsgemässen Zusammensetzung vermieden. Die erfindungsgemässe "Multiple unit"-Arzneidosierungsform vermeidet somit schon aus diesem Grunde und ferner wegen ihrer besonderen Art der Retardierung, dass signifikante Wirkstoffmengen bereits im Magen freigesetzt werden könnten, weshalb auf eine magensaftresistente Umhüllung verzichtet werden kann. Sie gestattet daher auch ohne enterischen Überzug eine gezielte und zudem pH-unabhängige Freigabesteuerung über bis zu 8 Stunden oder gewünschtenfalls auch über einen längeren Zeitraum.

In der erfindungsgemässen Zusammensetzung erfolgt die Retardierung durch eine Kombination von mindestens drei Massnahmen, von denen jede zur Verzögerung der Wirkstofffreisetzung beträgt, nämlich durch Mischen des Wirkstoffes mit einem magen- und darmsaftunlöslichen Polymer (d.h. durch Bildung einer Teilchenmatrix), durch die geringe Porengrösse, was eine entsprechende Verdichtung des Kernmaterials bedingt, und durch Umhüllung mit einem magen- und darmsaftunlöslichen Polymer. Diese Methode hat unter anderem den Vorteil, dass die Retardierung von der Form und Grösse der Teilchen weitgehend unabhängig ist und dass daher auch nicht-sphärische Teilchen oder Teilchen unterschiedlicher Grösse verwendet werden können. Zudem hat sich gezeigt, dass auf diese Weise eine sehr effiziente Retardierung schon mit geringen Mengen an unlöslichen Polymeren möglich ist und daher Retardformulierungen mit sehr hohem Wirkstoffanteil von bis zu etwa 97 Gew.-%möglich sind. Ferner ist die erfindungsgemässe Art der Retardierung nicht von einer allfälligen äusseren Phase (z.B. Tablettierhilfsstoffen) abhängig, und die Retardierung der Teilchen wird im Gegensatz zu vorbekannten Formulierungen auch durch ein Verpressen zu Tabletten nicht signifikant beeinträchtigt, da die erfindungsgemäss verwendeten, hochverdichteten, lackierten Matrixteilchen mechanisch sehr stabil sind. Die erfindungsgemässe Art der Retardierung hat überdies den Vorteil, dass perfekt teilbare Arzneiformen, beispielsweise teilbare Retardtabletten (z.B. mit Sollbruchrille), möglich sind, da die Retardierung durch die Teilung nicht beeinflusst wird. Weiterhin wurde gefunden, die erfindungsgemässen Zusammensetzungen durch Alterung und Temperaturschwankungen weniger beeinflusst werden und daher auch nach längerer Lagerdauer keine signifikanten Änderungen in den Freisetzungseigenschaften zu beobachten sind.

Die vorliegende Erfindung gestattet daher die Herstellung verbesserter Retardformen, die zudem kostengünstig und mit hoher Reproduzierbarkeit erhalten werden können.

Die erfindungsgemässe Formulierung eignet sich zur Verabreichung grundsätzlich beliebiger pharmazeutischer Wirkstoffe, die vorzugsweise im Darm und/oder Colon freigesetzt werden sollen, und insbesondere solcher, die mit Vorteil in retardierter Form verabreicht werden können, wie Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele geeigneter Wirkstoffe sind Acarbose, Betarezeptorenblocker, Nichtsteroidale Antirheumatia, Herzglykoside, Acetylsalicylsäure, Virustatika, Aclarubicin, Acyclovir, Cisplatin, Actinomycin, alpha- und beta-Sympatomimetika, Omeprazol, Allopurinol, Alprostadil, Prostaglandine, Amantadin, Ambroxol, Amlodipin, Methotrexat, 5-Aminosalicylsäure, Amitriptylin, Amoxicillin, Anastrozol, Atenolol, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridamol, Domperidon und Domperidonderivate, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolderivate, Itraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat, Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate, Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Silikate, Simvastatin, Sitosterin, Sotalol, Spagluminsäure, Sparfloxacin, Spectinomycin, Spiramycin, Spirapril, Spironoiacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsäurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Antiöstrogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valproinsäure, Vancomycin, Vecuroniumchlorid, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zidovudin, Zolmitriptan, Zolpidem, Zoplicon, Zotepin und dergleichen.

Beispiele besonders bevorzugter Wirkstoffe sind Analgetika, wie Tramadol oder Morphin, Mittel zur Behandlung von Colitis ulcerosa oder Morbus Crohn, wie 5-Aminosalicylsäure, Corticosteroide, wie Budesonid, Protonenpumpen-Inhibitoren, wie Omeprazol, Virusstatika, wie Acyclovir, Lipidsenker, wie Simvastatin oder Pravastatin, H2-Blocker, wie Ranitidin oder Famotidin, Antibiotika, wie Amoxicillin und/oder Clavulansäure, und ACE-Hemmer, wie Enalapril oder Amlodipin.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

Das im Gemisch mit dem Wirkstoff, d.h. im Kern der überzogenen Teilchen vorhandene Polymer kann grundsätzlich ein beliebiges, in Magen- und Darmsaft im wesentlichen unlösliches Polymer sein, das sich zur Matrixretardierung eignet. Vorzugsweise kann ein Polymer verwendet werden, das im Magen- und/oder Darmsaft quellbar und/oder erodierbar ist. Geeignete Materialien, beispielsweise Celluloseether wie Ethylcellulose, Celluloseester wie Celluloseacetat, und insbesondere Polymere und Copolymere von Acryl- und/oder Methacrylsäureestern, sind dem Fachmann bekannt. Bevorzugt sind in der Regel Polymere mit vergleichsweise geringer Permeabilität. Besonders bevorzugt sind Copolymerisate aus Acryl- und Methacrylsäureestern, in denen die Esterreste vorzugsweise Methyl- und Ethylgruppen sein können; vorzugsweise können sie einen geringen Gehalt an quartären Ammoniumgruppen von bis zu etwa 1:20 im molaren Verhältnis zu den übrigen neutralen (Meth)acrylsäureestern aufweisen. Beispiele besonders geeigneter Polymere sind Eudragit® NE und insbesondere Eudragit® RS (Rohm & Haas, Japan). Gewünschtenfalls kann auch ein Gemisch zweier oder mehrere solcher Polymere verwendet werden. Gewünschtenfalls kann das Gemisch aus Wirkstoff und magen- und darmsaftunlöslichem Polymer auch Polymere, die in verdünnten Säuren und/oder bei neutralem pH löslich sind, oder andere Hilfsstoffe enthalten, um die Freisetzungseigenschaften zu modifizieren. Als Zusätze eignen sich beispielsweise Eudragit® E, Eudragit® L, Eudragit® S (Rohm & Haas, Japan) sowie Schellack, Polyethylenglykole, Weichmacher und wasserlösliche Polymere, wie Chitosane. Im allgemeinen ist es bevorzugt, höchstens bis zu etwa 35 Gew.-%, bezogen auf den wirkstoffhaltigen Kern, an solchen Zusätzen zu verwenden, wobei der Anteil solcher Zusätze - falls vorhanden - beispielsweise etwa 1-20 Gew.-% betragen kann. Dementsprechend kann der wirkstoffhaltige Kern Wirkstoff und magen- und darmsaftunlösliches Polymer in einer Gesamtmenge von vorzugsweise mindestens etwa 65 Gew.-%, beispielsweise etwa 80-99 Gew.-%, enthalten oder insbesondere auch ausschliesslich aus Wirkstoff und magen- und darmsaftunlöslichem Polymer bestehen.

Die Menge an magen- und darmsaftunlöslichem Polymer im Kern der überzogenen Teilchen, kann je nach gewünschter Retardierung und je nach verwendetem Polymer und Wirkstoff variieren. Die optimale Menge kann vom Fachmann fallweise leicht anhand einiger Versuche ermittelt werden. Im allgemeinen genügt jedoch bereits eine Polymermenge von etwa 2-30 Gew.-%, vorzugsweise etwa 4-15 Gew.-%, bezogen auf den Wirkstoff, bzw. etwa 2-18 Gew.-%, vorzugsweise etwa 4-14 Gew.-%, bezogen auf die überzogenen Teilchen, wobei aber auch höhere Mengen durchaus möglich sind.

Das im Überzug der umhüllten Teilchen vorhandene Polymer kann grundsätzlich ebenfalls ein beliebiges, in Magen- und Darmsaft im wesentlichen unlösliches Polymer sein, das sich als Überzugsmaterial zur Retardierung eignet. Vorzugsweise kann ein Polymer verwendet werden, das im Magenund/oder Darmsaft quellbar und/oder erodierbar ist. Geeignete Materialien, beispielsweise Celluloseether wie Ethylcellulose, Celluloseester wie Celluloseacetat, und insbesondere Polymere und Copolymere von Acryl- und/oder Methacrylsäureestern, sind dem Fachmann bekannt. Bevorzugt sind in der Regel Polymere mit vergleichsweise geringer Permeabilität. Besonders bevorzugt sind Copolymerisate aus Acrylund Methacrylsäureestern, in denen die Esterreste vorzugsweise Methyl- und Ethylgruppen sein können; gewünschtenfalls können sie einen geringen Gehalt an quartären Ammoniumgruppen von bis zu etwa 1:20 im molaren Verhältnis zu den übrigen neutralen (Meth)acrylsäureestern aufweisen, wobei aber meist solche Polymere bevorzugt sind, die keine Ammoniumgruppen enthalten. Beispiele besonders geeigneter Polymere sind Eudragit® RS und insbesondere Eudragit® NE (Rohm & Haas, Japan). Gewünschtenfalls können auch zwei oder mehrere solche Polymere im gleichen oder in separaten Überzügen verwendet werden. Gewünschtenfalls kann der Überzug neben magen- und darmsaftunlöslichem Polymer auch magensaftlösliche und/oder darmsaftlösliche Materialien beispielsweise Schellack, Polyethylenglykole, Chitosane oder vorzugsweise enterische Polymere, wie Eudragit® L oder Eudragit® S (Rohm & Haas, Japan), enthalten, um die Freisetzungseigenschaften zu modifizieren. Im allgemeinen ist es jedoch bevorzugt, höchstens bis zu etwa 35 Gew.-%, bezogen auf die Gesamtmenge an Überzugsmaterialien, an solchen Materialien zu verwenden, wobei der Anteil solcher Materialien - falls vorhanden - beispielsweise etwa 1-20 Gew.-% betragen kann. Dementsprechend kann der Überzug einen Anteil an magen- und darmsaftunlöslichem Polymer von vorzugsweise mindestens etwa 65 Gew.-%, beispielsweise etwa 80-99 Gew.-%, enthalten oder insbesondere auch ausschliesslich aus einem oder mehreren magen- und darmsaftunlöslichen Polymeren bestehen.

Die Menge an magen- und darmsaftunlöslichem Polymer im Überzug der umhüllten Teilchen, kann ebenfalls je nach gewünschter Retardierung und je nach verwendetem Polymer und Wirkstoff variieren. Die optimale Menge kann vom Fachmann fallweise leicht anhand einiger Versuche ermittelt werden. Im allgemeinen genügt jedoch bereits eine Polymermenge von etwa 2-30 Gew.-%, vorzugsweise etwa 4-15 Gew.-%, bezogen auf den Wirkstoff, bzw. etwa 2-18 Gew.-%, vorzugsweise etwa 4-14 Gew.-%, bezogen auf die überzogenen Teilchen, wobei aber auch höhere Mengen durchaus möglich sind.

Gewünschtenfalls kann der Kern und/oder der Überzug der umhüllten Teilchen übliche Hilfsstoffe als Zusätze enthalten, beispielsweise einen Weichmacher wie Triethylcitrat und/oder ein Gleitmittel wie Talkum und/oder Glycerinmonostearat. Vorzugsweise kann in diesen Fällen der Kern der umhüllten Teilchen einen Weichmacher, wie Triethylcitrat, in einer Menge von beispielsweise etwa 0,1 bis 3 Gew.-%, bezogen auf die umhüllten Teilchen, und/oder der Überzug ein Gleitmittel, wie Talkum, in einer Menge von beispielsweise etwa 0,1 bis 5 Gew.-%, bezogen auf die umhüllten Teilchen, enthalten.

Weitere bevorzugte Aspekte der erfindungsgemässen Zusammensetzung ergeben sich aus der folgenden Beschreibung ihrer Herstellung.

Die Erfindung betrifft ebenfalls ein Verfahren zur Herstellung der neuen Zusammensetzung, welches dadurch gekennzeichnet ist, dass man den pharmazeutischen Wirkstoff mit einem in Magen- und Darmsaft unlöslichen Polymer so vermischt und zu einer Masse kompaktiert, dass die kompaktierte Masse einen mittleren inneren Porendurchmesser von höchstens 35 µm, vorzugsweise höchstens 20 µm, besitzt, dass man die kompaktierte Masse zu Teilchen zerkleinert und die Teilchen mit einem in Magen- und Darmsaft unlöslichen Polymer überzieht, und dass man, gewünschtenfalls, die überzogenen Teilchen in eine geeignete Darreichungsform bringt.

Die einzelnen Verfahrensschritte können nach an sich bekannten Methoden durchgeführt werden. Vorzugsweise kann jedoch das Vermischen von Wirkstoff und magen- und darmsaftunlöslichem Polymer durch Granulation erfolgen, indem man den Wirkstoff, der z.B. in Form eines Pulvers vorliegen kann, mit einer Dispersion oder Lösung des Polymers (z.B. einer 30%igen wässrigen Dispersion von Eudragit® RS) befeuchtet und das Gemisch in an sich bekannter Weise granuliert und trocknet. Als Polymerdispersionen oder -lösungen eignen sich Dispersionen und Lösungen in Wasser und/oder organischen Lösungsmitteln. Allfällige weitere Zusätze oder Hilfsstoffe können je nach Art der Materialien entweder zusammen mit dem Wirkstoff befeuchtet oder als Lösung oder Dispersion zugegeben werden. Vorzugsweise kann die Granulierung unter hohem Energieeintrag, beispielsweise durch Rühren mit hoher Geschwindigkeit, erfolgen, um die Schüttdichte zu erhöhen, d.h. das Wirkstoff/Polymer-Gemisch zu verdichten. Hierzu eignen sich beispielsweise die bekannten Vakuumgranulatoren der Firmen Colette (Grossbritannien), Zanchetta (Italien) und Bohle (Deutschland). Vorzugsweise ist dabei der Energieeintrag so hoch, dass sich das Granulat während der z.B. etwa 10- bis 20-minütigen Befeuchtung um mindestens etwa 1°C, beispielsweise etwa 1-5°C, erwärmt. Die anschliessende Trocknung kann in an sich bekannter Weise, z.B. unter Vakuum oder mittels Konvektionstrocknung, erfolgen.

Die anschliessende Verdichtung des Gemisches (z.B. eines Granulates) aus Wirkstoff, magen- und darmsaftunlöslichem Polymer und allfälligen weiteren Zusätzen kann ebenfalls in an sich bekannter Weise, vorzugsweise mittels eines Walzenkompaktors wie beispielsweise einem Pharmapaktor L200/50P der Firma Bepex Hosokawa (Japan) oder einer Walzenpresse vom Typ 250/100/3 der Firma Gerteis (Schweiz), erfolgen. Die Spaltbreite der Walzen kann beispielsweise zwischen etwa 0,2 und 3,5 mm liegen. Vorzugsweise kann der anliegende Pressdruck bei der Kompaktierung mindestens etwa 5 kN, beispielsweise etwa 5-30 kN, pro cm Presslänge betragen. Ferner ist es im allgemeinen bevorzugt, bei der Kompaktierung einen höheren Pressdruck anzuwenden, als bei einer allfälligen späteren Tablettierung. Mit diesen Massnahmen können Poren mit einem inneren Porendurchmesser von mehr als 35 µm praktisch völlig eliminiert und das Wirkstoff/Polymer-Gemisch so stark verdichtet werden, dass dessen Dichte um mindestens 10%, häufig um 50% und mehr, über der Schüttdichte der Ausgangssubstanz liegt.

Das erhaltene Kompaktat, d.h. das verdichtete Wirkstoff/Polymer-Gemisch, kann anschliessend in an sich bekannter Weise auf die gewünschte Teilchengrösse, die vorzugsweise im Bereich von etwa 0,1 bis 3,0 mm liegen kann, zerkleinert werden. Gemäss einer bevorzugten Variante kann dies dadurch erfolgen, dass das Kompaktat über einen geeigneten Sieb, beispielsweise ein Rotationssieb, gebrochen und die Kompaktatteilchen auf die gewünschte Teilchengrösse eingestellt werden. Hierbei entstehen meist unregelmässig geformte, nicht-sphärische Teilchen. Wie bereits oben erwähnt, haben jedoch Form und Grösse der Teilchen der erfindungsgemässen Zusammensetzungen praktisch keinen Einfluss auf deren Freisetzungsverhalten, so dass keine separaten Massnahmen zur Bildung annähernd sphärischer Teilchen erforderlich sind. Die üblicherweise verwendete Kennzahl zur Umschreibung des Formfaktors von Partikeln ist die Sphärizität nach Wadell, welche das Verhältnis der Oberfläche der volmengleichen Kugel zu der tatsächlich ermittelten Oberfläche darstellt. Während ideal sphärische Partikel eine Sphärizität von 1 besitzen, können - im Gegensatz zu den vorbekannten Formulierungen - erfindungsgemäss ohne weiteres auch Teilchen mit einer Sphärizität von beispielsweise weniger als 0,9 oder auch weniger als 0,8 verwendet werden. Selbstverständlich ist eine höhere Sphärizität nicht nachteilig. Dennoch kann gesagt werden, dass eine hohe Sphärizität erfindungsgemäss nicht erforderlich ist und dass die erfindungsgemäss verwendeten überzogenen Teilchen meist mehrheitlich (d. h. zu über 50% der Teilchen) eine Sphärizität von weniger als 0,9 oder sogar von weniger als 0,8 aufweisen können.

Die Umhüllung der kompaktierten Teilchen mit einem in Magen- und Darmsaft unlöslichen Polymer kann ebenfalls in an sich bekannter Weise erfolgen, beispielsweise durch Trommel-Coating oder vorzugsweise durch Wirbelschicht-Coating. Vorzugsweise kann hierbei eine wässrige Dispersion des Polymers, beispielsweise eine 40%ige wässrige Dispersion von Eudragit® NE, verwendet werden. Gewünschtenfalls können dem Überzugsmaterial weitere Substanzen zugesetzt werden, beispielsweise Chitosan, ein enterisches Polymer, ein Gleitmittel wie Talkum oder ein Antischaummittel. Die Trocknung der überzogenen Teilchen kann bei üblichen Temperaturen und gegebenenfalls im Vakuum erfolgen.

Die erhaltenen überzogenen Teilchen können gewünschtenfalls in an sich bekannter Weise und unter Verwendung üblicher Tablettierhilfsstoffe, wie Bindemittel, Sprengmittel, Gleitmittel und dergleichen, zu Tabletten verarbeitet werden. Diese zeichnen sich u.a. dadurch aus, dass die Freisetzungsgeschwindigkeit im wesentlich unabhängig von dem bei der Tablettierung angewendeten Pressdruck und von der Härte der Tablette ist und dass sie ohne signifikante Veränderung des Freisetzungsverhaltens teilbar sind. Vorzugsweise kann bei der Tablettierung eine elastische, druckabsorbierende und im Magen rasch zerfallende äussere Tablettenphase verwendet werden. Retardtabletten, die im Magen rasch, z.B. innert weniger als 30 Sekunden, zerfallen, sind insbesondere dann erwünscht, wenn die Verweilzeit im Magen möglichst kurz sein soll. Als besonders geeignete Tablettierhilfsstoffe hat sich eine Kombination von mikrokristalliner Cellulose, wasserlöslichem Polyvinylpyrrolidon und quervernetztem wasserunlöslichem Polyvinylpyrrolidon erwiesen. Vorzugsweise kann hierbei die mikrokristalline Cellulose und das wasserlösliche Polyvinylpyrrolidon zuerst zu einem Hilfsgranulat verarbeitet und dann zusammen mit den überzogenen Teilchen und dem quervernetzten Polyvinylpyrrolidon zu Tabletten verpresst werden. Die Herstellung des Hilfsgranulates kann beispielsweise in einem Wirbelschichtgranulator WSG der Firma Glatt (Schweiz) oder einem Wirbelschichtgranulator HKC der Firma BWI (Deutschland) durchgeführt werden. Der Anteil an Tablettierhilfsstoffen, bezogen auf die Gesamtformulierung, kann beispielsweise etwa 3 bis 90 Gew.-% oder mehr, insbesondere etwa 20 bis 60 Gew.-%, betragen. Gewünschtenfalls kann der Anteil an Tablettierhilfsstoffen sehr niedrig gehalten werden, was insbesondere bei hoch dosierten Wirkstoffen von Vorteil ist, während bei niedrig dosierten Wirkstoffen in der Regel höhere Anteile an Hilfsstoffen von bis zu etwa 90 Gew.-% oder mehr verwendet werden, um eine übliche Tablettengrösse zu erhalten. Gewünschtenfalls können erfindungsgemäss Tabletten mit einem hohen Wirkstoffanteil von über 90 Gew.-% oder sogar über 95 Gew.-% und dennoch guter Retardierung erhalten werden. Gemäss einem weiteren bevorzugten Aspekt können die erfindungsgemäss erhältlichen überzogenen wirkstoffhaltigen Teilchen insbesondere auch mit weniger als 3 Gew.-% an Tablettierhilfsstoffen, bezogen auf die gesamte Tablettenformulierung, oder sogar völlig ohne Tabettierhilfsstoffe zu Tabletten verpresst werden. Eine dabei gelegentlich auftretende, geringfügige Verschlechterung der Retardierung kann ohne weiteres durch eine geringfügig höhere Menge an Überzug in den wirkstoffhaltigen Teilchen kompensiert werden.

Die überzogenen Teilchen können gewünschtenfalls auch als solche oral verabreicht oder in an sich bekannter Weise zu anderen Verabreichungsformen wie Dragées, Kapseln, Filmtabletten, Disperstabletten, Lingualdisperstabletten, Brausetabletten, Sachets, Trockensäfte, Suppositorien und dergleichen verarbeitet werden.

Die Erfindung wird durch die folgenden Beispiele weiter veranschaulicht. Als Antischaumemulsion wurde jeweils Simethicone Emulsion USP, enthaltend 28,5 Gew.-% Dimethicon (ein Siliconöl), 1,5 Gew.-% Kieselsäure, 3 Gew.-% Methylcellulose, 0,1 Gew.-% Sorbinsäure und 66,9 Gew.-% Wasser, verwendet (Angaben betreffend die Zusammensetzung der Gesamtformulierungen beziehen sich jeweils nur auf den Feststoffanteil). Eudragit® RS30D ist eine 30%ige wässrige Dispersion von Eudragit® RS, und Eudragit® NE40D ist eine 40%ige wässrige Dispersion von Eudragit® NE (Rohm & Hass, Japan). Eudragit® E 12,5 ist eine 12,5%ige Lösung von Eudragit® E in Isopropanol/Aceton (60:40), und Eudragit® S 12,5 ist eine 12,5%ige Lösung von Eudragit® S in Isopropanol (Rohm & Hass, Japan). Kollidon K90 (Hoechst, Deutschland) ist ein Polyvinylpyrrolidon mit einem Molekulargewicht von etwa 90'000. Kollidon CL (Hoechst, Deutschland) ist ein quervernetztes wasserunlösliches Polyvinylpyrrolidon.

Beispiel 1

Tablettenformulierung von 5-Aminosalicylsäure, enthaltend pro Tablette:

| | |
|---|---|
| 5-Aminosalicylsäure | 500,00 mg |
| Eudragit RS | 25,00 mg |
| Triethylcitrat | 5,00 mg |
| Kompaktat-Teilchen insgesamt | 530,00 mg |
| Eudragit NE | 23,85 mg |
| Talkum | 12,67 mg |
| Simethicone Emulsion USP | 0,48 mg |
| überzogene Teilchen insgesamt | 567,00 mg |
| mikrokristalline Cellulose | 144,06 mg |
| Kollidon K90 | 8,94 mg |
| Kollidon CL | 40,00 mg |
| Tablette insgesamt | 760,00 mg |

Zur Herstellung von 350'000 Tabletten werden 175 kg 5-Aminosalicylsäure in einem 400 1-Vakuumgranulator Roto P/F der Firma Zanchetta (Italien) innert 10-20 Minuten mit einer wässrigen Dispersion aus 29,167 kg Eudragit RS30D (enthaltend 8,750 kg Eudragit RS), 1,750 kg Triethylcitrat und 7,65 kg Wasser befeuchtet und unter hohem Energieeintrag (durch Rühren mit hoher Geschwindigkeit, wobei sich das Gemisch um 4°C erwärmt) verdichtet. Das erhaltene Granulat wird anschliessend mittels Konvektionstrocknung bei 50-90°C getrocknet, bis der Restwassergehalt kleiner als 1 Gew.-% ist. Danach wird das getrocknete Granulat mittels einer Walzenpresse Typ 250/100/3 der Firma Gerteis (Schweiz) bei einem anliegenden Pressdruck von 15-20 kN pro cm Presslänge und einer Spaltbreite der Walzen von 2,0 ± 0,5 mm weiter verdichtet. Die bei der Kompaktierung erhaltene Schülpe wird über ein Rotationssieb gebrochen und das entstandene Kompaktat auf eine Teilchengrösse von 0,6-1,25 mm eingestellt.

Dieses fraktionierte Kompaktat wird anschliessend mit einer wässrigen Suspension aus 20,869 kg Eudragit NE40D (enthaltend 8,348 kg Eudragit NE), 4,435 kg Talkum, 509,0 g einer 33%igen Antischaumemulsion (Simethicone Emulsion USP) und 20,867 kg Wasser lackiert. Um die elektrostatische Aufladung bei diesem Vorgang möglichst gering zu halten, wird periodisch weiteres Talkum (ingesamt 2,765 kg) auf das Kompaktat gegeben.

In einem Wirbelschichtgranulator vom Typ HKC der Firma BWI (Deutschland) werden 50,421 kg mikrokristalline Cellulose mit einer Lösung aus 3,129 kg Kollidon K90 in 35,000 kg Wasser granuliert. Anschliessend wird das lackierte und fraktionierte Kompaktat (198,450 kg) mit dem erhaltenen Granulat (53,550 kg) und 14,000 kg Kollidon CL vermischt und unter einem Druck von 25-45 kN zu runden Tabletten mit einem Durchmesser von 13,5 mm, einer Höhe von 4,5 mm und einer Masse von 760 mg verpresst. Die erhaltenen Tabletten haben eine Härte von mehr als 0,8 N pro mm$^2$ Bruchfläche.

Beispiel 2

Zur Untersuchung des Freisetzungsverhaltens von in analoger Weise zu Beispiel 1 hergestellten Tabletten von 5-Aminosalicylsäure wurden die nachfolgend beschriebenen Versuche durchgeführt. Die Wirkstofffreisetzung wurde jeweils mit einem Sotax AT7 (Paddle-Methode) der Firma Sotax (Schweiz) gemäss Europäischer Pharmakopöe und US-Pharmakopöe gemessen.

a) In zwei Chargen erhaltene Tabletten mit einer maximalen Teilchengrösse der überzogenen Wirkstoffteilchen von 1000 µm (Charge I) bzw. 700 µm (Charge II) wurden bei pH 1,2 (0,1 N Salzsäure) auf ihre Freisetzungsgeschwindigkeit untersucht. Die in Tabelle 1 zusammengestellten Ergebnisse zeigen, dass die Teilchengrösse praktisch keinen Einfluss auf Freigabewerte der erfindungsgemässen Formulierung hat, während die Freisetzung aus herkömmlichen überzogenen Granulaten in der Regel von der Oberfläche und der Teilchengrösse abhängig ist.

Tabelle 1

| Zeit [min.] | Freigabe [%] | |
|---|---|---|
| | Charge I (1000 µm) | Charge II (700 µm) |
| 30 | 24,9 | 25,3 |
| 60 | 38,8 | 38,9 |
| 90 | 49,7 | 49,7 |
| 120 | 58,8 | 58,3 |

Tabelle 1   (fortgesetzt)

| Zeit [min.] | Freigabe [%] | |
|---|---|---|
| | Charge I (1000 µm) | Charge II (700 µm) |
| 150 | 66,5 | 65,7 |
| 180 | 72,9 | 72,0 |
| 210 | 78,2 | 77,4 |
| 240 | 82,5 | 81,7 |

b) Tabletten gemäss Charge I von Absatz a) wurden 24 h bei 50°C bzw. 65 h bei 60°C getempert und dann deren Wirkstofffreigabe im Vergleich zu ungetemperten Tabletten in ICH-Phosphatpuffer pH 6,8 untersucht. Wie die in Tabelle 2 zusammengestellten Ergebnisse zeigen, wird die Wirkstofffreigabe auch von der Art und den Bedingungen der Temperung fast nicht beeinflusst.

Tabelle 2

| Zeit [min.] | Freigabe [%] | | |
|---|---|---|---|
| | ungetempert | 24 h / 50°C | 65 h / 60°C |
| 30 | 19,2 | 18,7 | 19,0 |
| 60 | 34,5 | 33,4 | 34,0 |
| 90 | 47,2 | 45,6 | 46,4 |
| 120 | 57,6 | 55,7 | 56,7 |
| 150 | 66,2 | 64,2 | 65,2 |
| 180 | 73,0 | 71,1 | 72,1 |
| 210 | 78,6 | 76,9 | 77,8 |
| 240 | 82,8 | 81,2 | 82,1 |

c) Tabletten gemäss Charge I von Absatz a) wurden halbiert und dann deren Wirkstofffreigabe im Vergleich zu ganzen Tabletten bei pH 1,2 (0,1 N Salzsäure) untersucht. Die in Tabelle 3 zusammengestellten Ergebnisse zeigen, dass die Retardierung durch die Halbierung der Tabletten nicht beeinträchtigt wird.

Tabelle 3

| Zeit [min.] | Freigabe [%] | |
|---|---|---|
| | ganze Tablette | halbe Tablette |
| 30 | 24,9 | 24,0 |
| 60 | 38,8 | 38,2 |
| 90 | 49,7 | 49,0 |
| 120 | 58,8 | 58,1 |
| 150 | 66,5 | 65,7 |
| 180 | 72,9 | 71,8 |
| 210 | 78,2 | 77,4 |
| 240 | 82,5 | 81,4 |

d) In analoger Weise zu Beispiel 1 wurden 3 Chargen von 5-Aminosalicylsäure-Tabletten mit einer Tablettenhärte von 80 N, 120 N bzw. 170 N (gemäss Härtetestgerät der Firma Kraemer, Deutschland) hergestellt. Wie die in Tabelle 4 zusammengestellten Freigabewerte in ICH-Phosphatpuffer pH 6,8 zeigen, wird die Freisetzungsgeschwindigkeit auch durch die Tablettenhärte kaum beeinflusst.

Tabelle 4

| Zeit [min.] | Freigabe [%] | | |
|---|---|---|---|
| | 80 N | 120 N | 170 N |
| 30 | 17,5 | 18,7 | 19,4 |

Tabelle 4   (fortgesetzt)

| Zeit [min.] | Freigabe [%] | | |
|---|---|---|---|
| | 80 N | 120 N | 170 N |
| 60 | 32,5 | 33,4 | 34,0 |
| 90 | 46,2 | 45,6 | 47,4 |
| 120 | 55,6 | 55,7 | 57,7 |
| 150 | 65,2 | 64,2 | 65,2 |
| 180 | 71,0 | 71,1 | 72,1 |
| 210 | 75,5 | 76,9 | 76,8 |
| 240 | 81,8 | 81,2 | 82,0 |

e) In analoger Weise zu Beispiel 1 wurden 2 Chargen von 5-Aminosalicylsäure-Tabletten hergestellt, wobei aber 25 Gew.-% bzw. 50 Gew.-% an äusseren Tablettierhilfsstoffen, bezogen auf die gesamte Tablettenformulierung, verwendet wurden. Die in Tabelle 5 zusammengestellten Freigabewerte bei pH 1,2 (0,1 N Salzsäure) zeigen, dass die Wirkstofffreisetzung auch durch die Menge an Tablettierhilfsstoffen kaum beeinflusst wird.

Tabelle 5

| Zeit [min.] | Freigabe [%] | |
|---|---|---|
| | 25% Tablettierhilfsstoffe | 50% Tablettierhilfsstoffe |
| 30 | 24,9 | 23,8 |
| 60 | 38,8 | 37,2 |
| 90 | 49,7 | 48,1 |
| 120 | 58,8 | 57,5 |
| 150 | 66,5 | 66,0 |
| 180 | 72,9 | 71,9 |
| 210 | 78,2 | 77,4 |
| 240 | 82,5 | 82,4 |

Beispiel 3

Tablettenformulierung von Tramadol-Hydrochlorid, enthaltend pro Tablette:

| Tramadol-Hydrochlorid | 100,00 mg |
|---|---|
| Eudragit RS | 10,00 mg |
| Triethylcitrat | 2,00 mg |
| Kompaktat-Teilchen insgesamt | 112,00 mg |
| Eudragit NE | 5,60 mg |
| Talkum | 2,00 mg |
| Simethicone Emulsion USP | 0,66 mg |
| überzogene Teilchen insgesamt | 120,26 mg |
| mikrokristalline Cellulose | 170,00 mg |
| Kollidon K90 | 14,74 mg |
| Kollidon CL | 15,00 mg |
| Tablette insgesamt | 320,00 mg |

Zur Herstellung von 350'000 Tabletten werden 35,0 kg Tramadol-Hydrochlorid in analoger Weise zu Beispiel 1 mit einer wässrigen Dispersion aus 11,67 kg Eudragit RS30D (enthaltend 3,5 kg Eudragit RS), 700 g Triethylcitrat und ca. 2,0 kg Wasser befeuchtet, granuliert, bei 80°C getrocknet, kompaktiert und fraktioniert (Pressdruck 15-35 kN/cm, Teilchengrösse 0,6-1,25 mm). Auf dieses Kompaktat wird in analoger Weise zu Beispiel 1 unter Verwendung von 4,9 kg Eudragit NE40D (enthaltend 1,96 kg Eudragit NE), 700 g Talkum, 700g einer 33%igen Simethicone Emulsion USP und 4,4 kg Wasser ein Lacküberzug aufgebracht. Anschliessend wird dieses überzogene Kompaktat in analoger Weise zu Beispiel 1 mit 5,250 kg Kollidon CL und einem Granulat aus 59,5 kg mikrokristalliner Cellulose und 5,166 kg Kollidon K90 gemischt und zu Tabletten mit einer Masse von 320 mg verpresst.

In analoger Weise können 420 mg-Tabletten mit einem höheren Gehalt von z.B. 200 mg an Tramadol-Hydrochlorid unter Verwendung einer entsprechend geringeren Menge an Granulat aus mikrokristalliner Cellulose und Kollidon K90 bei der Tablettierung erhalten werden.

Beispiel 4

Tablettenformulierung von Morphin-Hydrochlorid, enthaltend pro Tablette:

| | |
|---|---|
| Morphin-Hydrochlorid | 20,00 mg |
| Eudragit RS | 5,00 mg |
| Eudragit E | 0,50 mg |
| Triethylcitrat | 1,00 mg |
| Kompaktat-Teilchen insgesamt | 26,50 mg |
| Eudragit NE | 5,00 mg |
| Talkum | 1,00 mg |
| Simethicone Emulsion USP | 0,33 mg |
| Eudragit S | 1,00 mg |
| überzogene Teilchen insgesamt | 33,83 mg |
| mikrokristalline Cellulose | 51,50 mg |
| Kollidon K90 | 5,67 mg |
| Kollidon CL | 9,00 mg |
| Tablette insgesamt | 100,00 mg |

Die Herstellung erfolgt in analoger Weise zu den Beispielen 1 und 2, wobei aber bei der Granulierung zum Gemisch aus Morphin-Hydrochlorid, Eudragit RS und Triethylcitrat noch Eudragit E 12,5 zugesetzt und nach der Lakkierung mit Eudragit NE, Talkum und Simethicone Emulsion die überzogenen wirkstoffhaltigen Teilchen noch mit Eudragit S 12,5 besprüht werden.

Beispiel 5

Tablettenformulierung von 5-Aminosalicylsäure, enthaltend pro Tablette:

| | |
|---|---|
| 5-Aminosalicylsäure | 750,00 mg |
| Eudragit RS | 37,50 mg |
| Triethylcitrat | 7,50 mg |
| Kompaktat-Teilchen insgesamt | 795,00 mg |
| Eudragit NE | 31,80 mg |
| Talkum | 15,56 mg |
| Simethicone Emulsion USP | 0,64 mg |
| überzogene Teilchen insgesamt | 843,00 mg |
| Kollidon CL | 50,00 mg |
| Tablette insgesamt | 893,00 mg |

Die Herstellung erfolgt in analoger Weise zu Beispiel 1. In analoger Weise können auch Tabletten ohne Tablettierhilfsstoffe, d.h. ohne Verwendung von Kollidon CL bei der Tablettierung, hergestellt werden.

**Patentansprüche**

1.   Pharmazeutische Zusammensetzung zur langsamen Freisetzung von Wirkstoff im Magen-Darm-Trakt, umfassend mehrere überzogene wirkstoffhaltige Teilchen, die einen wirkstoffhaltigen Kern und einen Überzug, umfassend ein in Magenund Darmsaft unlösliches Polymer, aufweisen, wobei der wirkstoffhaltige Kern der überzogenen Teilchen ein homogenes Gemisch, umfassend einen pharmazeutischen Wirkstoff und ein in Magen- und Darmsaft unlösliches Polymer, ist und einen mittleren inneren Porendurchmesser, gemessen mittels Quecksilberporosimetrie bei 1000 bis 4000 bar, von höchstens 35 μm besitzt.

**2.** Pharmazeutische Zusammensetzung zur langsamen Freisetzung von Wirkstoff im Magen-Darm-Trakt, umfassend mehrere überzogene wirkstoffhaltige Teilchen, die einen wirkstoffhaltigen Kern und einen Überzug, umfassend ein in Magenund Darmsaft unlösliches Polymer, aufweisen, wobei der wirkstoffhaltige Kern der überzogenen Teilchen ein homogenes Gemisch, umfassend einen pharmazeutischen Wirkstoff und ein in Magen- und Darmsaft unlösliches Polymer, ist und eine prozentuale Porosität von höchstens 27% aufweist.

**3.** Zusammensetzung nach Anspruch 1 oder 2, **dadurch gekennzeichnet, dass** das im Kern der überzogenen wirkstoffhaltigen Teilchen und/oder das im Überzug der überzogenen wirkstoffhaltigen Teilchen vorhandene Polymer ein im Magenund/oder Darmsaft quellbares und/oder erodierbares Polymer ist.

**4.** Zusammensetzung nach einem der Ansprüche 1 bis 3, **dadurch gekennzeichnet, dass** das im Kern der überzogenen wirkstoffhaltigen Teilchen und/oder das im Überzug der überzogenen wirkstoffhaltigen Teilchen vorhandene Polymer ein Celluloseether, ein Celluloseester oder ein Polymer oder Copolymer aus Acryl- und/oder Methacrylsäureestern, vorzugsweise ein Copolymer aus Acryl- und Methacrylsäureestern, ist.

**5.** Zusammensetzung nach einem der Ansprüche 1 bis 4, **dadurch gekennzeichnet, dass** der Kern der überzogenen wirkstoffhaltigen Teilchen 2-30 Gew.-% an magen- und darmsaftunlöslichem Polymer, bezogen auf den Wirkstoff, und/oder der Überzug der überzogenen wirkstoffhaltigen Teilchen 2-30 Gew.-% an magen- und darmsaftunlöslichem Polymer, bezogen auf den Wirkstoff, enthält.

**6.** Zusammensetzung nach einem der Ansprüche 1 bis 5, **dadurch gekennzeichnet, dass** die überzogenen wirkstoffhaltigen Teilchen eine Teilchengrösse von 0,1 bis 3,0 mm, vorzugsweise 0,2 bis 2,5 mm, aufweisen.

**7.** Zusammensetzung nach einem der Ansprüche 1 bis 6, **dadurch gekennzeichnet, dass** die überzogenen Teilchen mehrheitlich eine Sphärizität nach Wadell von weniger als 0,9 aufweisen.

**8.** Zusammensetzung nach einem der Ansprüche 1 bis 7, **dadurch gekennzeichnet, dass** der pharmazeutische Wirkstoff ein Wirkstoff aus der Gruppe der Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diurecika, Enzyme, Enzyminhibitoren, Gichtmittel, Hormone und deren Hemmstoffe, Herzglykoside, Immuntherapeutika und Zytokine, Laxantien, Lipidsenker, Migränemittel, Mineralstoffpräparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren ist.

**9.** Zusammensetzung nach einem der Ansprüche 1 bis 8, **dadurch gekennzeichnet, dass** der pharmazeutische Wirkstoff ein Wirkstoff aus der Gruppe der Analgetika, Mittel zur Behandlung von Colitis ulcerosa oder Morbus Crohn, Corticosteroide, Protonenpumpen-Inhibitoren, Virustatika, Lipidsenker, H2-Blocker, Antibiotika und ACE-Hemmer ist.

**10.** Zusammensetzung nach einem der Ansprüche 1 bis 9, **dadurch gekennzeichnet, dass** der phamzeutische Wirkstoff Tramadol, Morphin, 5-Aminosalicylsäure, Budesonid, Omeprazol, Acyclovir, Simvastatin, Pravastatin, Ranitidin, Famotidin, Amoxicillin, Clavulansäure, Enalapril, Amlodipin oder ein pharmazeutisch annehmbares Salz oder Derivat davon ist.

**11.** Zusammensetzung nach einem der Ansprüche 1 bis 10, in Form von Tabletten, Dragees, Kapseln, Filmtabletten, Disperstabletten, Lingualdisperstabletten, Brausetabletten, Sachets, Trockensäfte oder Suppositorien.

**12.** Zusammensetzung nach einem der Ansprüche 1 bis 11, in Form von Tabletten, enthaltend mikrokristalline Cellulose, wasserlösliches Polyvinylpyrrolidon und quervernetztes wasserunlösliches Polyvinylpyrrolidon als Tablettierhilfsstoffe.

**13.** Zusammensetzung nach einem der Ansprüche 1 bis 12 in Form einer teilbaren Retardtablette.

**14.** Verfahren zur Herstellung einer pharmazeutzschen Zusammensetzung gemäss einem der Ansprüche 1 und 3 bis 13, **dadurch gekennzeichnet, dass** man den pharmazeutischen Wirkstoff mit einem in Magen- und Darmsaft unlöslichen Polymer so vermischt und zu einer Masse kompaktiert, dass die kompaktierte Masse einen mittleren inneren Porendurchmesser, gemessen mittels Quecksilberporosimetrie bei 1000 bis 4000 bar, von höchstens 35 µm besitzt, dass man die kompaktierte Masse zu Teilchen zerkleinert und die Teilchen mit einem in Magen- und Darmsaft unlöslichen Polymer überzieht, und dass man, gewünschtenfalls, die überzogenen Teilchen in eine geeignete Darreichungsform bringt.

**15.** Verfahren zur Herstellung einer pharmazeutischen Zusammensetzung gemäss einem der Ansprüche 2 bis 13, **dadurch gekennzeichnet, dass** man den pharmazeutischen Wirkstoff mit einem in Magen- und Darmsaft unlöslichen Polymer so vermischt und zu einer Masse kompaktiert, dass die kompaktierte Masse eine prozentuale Porosität von höchstens 27% aufweist, dass man die kompaktierte Masse zu Teilchen zerkleinert und die Teilchen mit einem in Magen- und Darmsaft unlöslichen Polymer überzieht, und dass man, gewünschtenfalls, die überzogenen Teilchen in eine geeignete Darreichungsform bringt.

**16.** Verfahren nach Anspruch 14 oder 15, **dadurch gekennzeichnet, dass** man zum Vermischen des pharmazeutischen Wirkstoffes mit dem in Magne- und Darmsaft unlöslichen Polymer den Wirkstoff mit einer wässrigen und/oder organischen Dispersion bzw. Lösung des Polymers befeuchtet und das Gemisch granuliert und trocknet.

**17.** Verfahren nach einem der Ansprüche 14 bis 16, **dadurch gekennzeichnet, dass** die Lompaktierung unter einem Pressdruck von mindestens 5 kN pro cm Presslänge erfolgt.

## Claims

**1.** Pharmaceutical composition for slow release of active ingredient in the gastrointestinal tract, comprising a plurality of coated active ingredient-containing particles which have an active ingredient-containing core and a coating comprising a polymer insoluble in gastric and intestinal juices, where the active ingredient-containing core of the coated particles is a homogeneous mixture comprising an active pharmaceutical ingredient and a polymer insoluble in gastric and intestinal juices, and has an average internal pore diameter, measured by mercury porosimetry at 1000 to 4000 bar, not exceeding 35 µm.

**2.** Pharmaceutical composition for slow release of active ingredient in the gastrointestinal tract, comprising a plurality of coated active ingredient-containing particles which have an active ingredient-containing core and a coating comprising a polymer insoluble in gastric and intestinal juices, where the active ingredient-containing core of the coated particles is a homogeneous mixture comprising an active pharmaceutical ingredient and a polymer insoluble in gastric and intestinal juices, and has a percent porosity not exceeding 27%.

**3.** Composition as claimed in claim 1 or 2, wherein the polymer present in the core of the coated active ingredient-containing particles and/or the polymer present in the coating of the coated active ingredient-containing particles is a polymer which is able to swell and/or be eroded in gastric and/or intestinal juices.

**4.** Composition as claimed in any of claims 1 to 3, wherein the polymer present in the core of the coated active ingredient-containing particles and/or the polymer present in the coating of the coated active ingredient-containing particles is a cellulose ether, a cellulose ester or a polymer or copolymer of acrylic and/or methacrylic esters, preferably a copolymer of acrylic and methacrylic esters.

**5.** Composition as claimed in any of claims 1 to 4, wherein the core of the coated active ingredient-containing particles contains 2-30% by weight of polymer insoluble in gastric and intestinal juices, based on the active ingredient, and/or the coating of the coated active ingredient-containing particles contains 2-30% by weight of polymer insoluble in gastric and intestinal juices, based on the active ingredient.

**6.** Composition as claimed in any of claims 1 to 5, wherein the coated active ingredient-containing particles have a particle size of from 0.1 to 3.0 mm, preferably 0.2 to 2.5 mm.

**7.** Composition as claimed in any of claims 1 to 6, wherein the majority of the coated particles have a sphericity according to Wadell of less than 0.9.

**8.** Composition as claimed in any of claims 1 to 7, wherein the active pharmaceutical ingredient is an active ingredient from the group of antidiabetics, analgesics, antiinflammatory agents, antirheumatic agents, antihypotensives, antihypertensives, psychopharmaceuticals, tranquilizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, enzymes, enzyme inhibitors, gout remedies, hormones and their inhibitors, cardiac glycosides, immunotherapeutics and cytokines, laxatives, lipid-lowering agents, migraine remedies, mineral preparations, otologicals, antiparkinson agents, thyroid therapeutics, spasmolytics, platelet aggregation inhibitors, vitamins, cytostatics and metastasis inhibitors, phytopharmaceuticals, chemotherapeutics and amino acids.

**9.** Composition as claimed in any of claims 1 to 8, wherein the active pharmaceutical ingredient is an active ingredient from the group of analgesics, agents for treating ulcerative colitis or Crohn's disease, corticosteroids, proton pump inhibitors, virostatic agents, lipid-lowering agents, H2 blockers, antibiotics and ACE inhibitors.

**10.** Composition as claimed in any of claims 1 to 9, wherein the active pharmaceutical ingredient is tramadol, morphine, 5-aminosalicylic acid, budesonide, omeprazole, acyclovir, simvastatin, pravastatin, ranitidine, famotidine, amoxicillin, clavulanic acid, enalapril, amlodipine or a pharmaceutically acceptable salt or derivative thereof.

**11.** Composition as claimed in any of claims 1 to 10, in the form of tablets, sugar-coated tablets, capsules, film-coated tablets, disperse tablets, lingual disperse tablets, effervescent tablets, sachets, powders for reconstitution or suppositories.

**12.** Composition as claimed in any of claims 1 to 11, in the form of tablets containing microcrystalline cellulose, watersoluble polyvinylpyrrolidone and crosslinked water-insoluble polyvinylpyrrolidone as tablet excipients.

**13.** Composition as claimed in any of claims 1 to 12 in the form of a divisible delayed release tablet.

**14.** Process for producing a pharmaceutical composition as claimed in any of claims 1 and 3 to 13, which comprises the active pharmaceutical ingredient being mixed with a polymer insoluble in gastric and intestinal juices and compacted to a composition in such a way that the compacted composition has an average internal pore diameter, measured by mercury porosimetry at 1000 to 4000 bar, not exceeding 35 µm, and comprises the compacted composition being comminuted to particles, and the particles being coated with a polymer insoluble in gastric and intestinal juices, and comprises, if desired, the coated particles being converted into a suitable dosage form.

**15.** Process for producing a pharmaceutical composition as claimed in any of claims 2 to 13, which comprises the active pharmaceutical ingredient being mixed with a polymer insoluble in gastric and intestinal juices and compacted to a composition in such a way that the compacted composition has a percent porosity not exceeding 27%, and comprises the compacted composition being comminuted to particles, and the particles being coated with a polymer insoluble in gastric and intestinal juices, and comprises, if desired, the coated particles being converted into a suitable dosage form.

**16.** Process as claimed in claim 14 or 15, wherein for mixing the active pharmaceutical ingredient with the polymer insoluble in gastric and intestinal juices the active ingredient is moistened with an aqueous and/or organic dispersion or solution of the polymer, and the mixture is granulated and dried.

**17.** Process as claimed in any of claims 14 to 16, wherein the compaction takes place under a pressure of at least 5 kN per cm length of press.

**Revendications**

**1.** Composition pharmaceutique permettant la libération prolongée de substance active dans le tractus gastro-intestinal, comprenant plusieurs particules enrobées contenant de substance active, lesquelles comportent un noyau contenant la substance active et un enrobage comprenant un polymère insoluble dans le suc gastrique et le suc intestinal, le noyau des particules enrobées contenant la substance active étant un mélange homogène comprenant une substance active pharmaceutique et un polymère insoluble dans le suc gastrique et le suc intestinal, et présentant un diamètre de pores intérieur moyen de 35 µm au maximum, mesuré au moyen de la porosimétrie au mercure à une pression comprise entre 1 000 et 4 000 bars.

**2.** Composition pharmaceutique permettant la libération prolongée de substance active dans le tractus gastro-intestinal, comprenant plusieurs particules enrobées contenant de substance active, lesquelles comportent un noyau contenant la substance active et un enrobage comprenant un polymère insoluble dans le suc gastrique et le suc intestinal, le noyau des particules enrobées contenant la substance active étant un mélange homogène comprenant une substance active pharmaceutique et un polymère insoluble dans le suc gastrique et le suc intestinal, et présentant un pourcentage de porosité de 27 % au maximum.

**3.** Composition selon la revendication 1 ou 2, **caractérisée en ce que** le polymère présent dans le noyau des particules enrobées contenant la substance active et/ou le polymère présent dans l'enrobage des particules enrobées contenant la substance active est un polymère susceptible de gonfler et/ou de se dégrader dans le suc gastrique et/ou le suc intestinal.

**4.** Composition selon l'une quelconque des revendications 1 à 3, **caractérisée en ce que** le polymère présent dans le noyau des particules enrobées contenant la substance active et/ou le polymère présent dans l'enrobage des particules enrobées contenant la substance active est un éther de cellulose, un ester de cellulose ou un polymère ou copolymère formé par des esters d'acide acrylique et/ou des esters d'acide méthacrylique, de préférence un copolymère d'esters d'acide acrylique et d'acide méthacrylique.

**5.** Composition selon l'une quelconque des revendications 1 à 4, **caractérisée en ce que** le noyau des particules enrobées contenant la substance active contient 2 à 30 % en poids de polymère insoluble dans le suc gastrique et le suc intestinal par rapport à la substance active, et/ou l'enrobage des particules enrobées contenant la substance active contient 2 à 30 % en poids de polymère insoluble dans le suc gastrique et le suc intestinal par rapport à la substance active.

**6.** Composition selon l'une quelconque des revendications 1 à 5, **caractérisée en ce que** les particules enrobées contenant la substance active ont une granulométrie de 0,1 à 3,0 mm, de préférence de 0,2 à 2,5 mm.

**7.** Composition selon l'une quelconque des revendications 1 à 6, **caractérisée en ce que** les particules enrobées présentent en pluralité une sphéricité selon Wadell inférieure à 0,9.

**8.** Composition selon l'une quelconque des revendications 1 à 7, **caractérisée en ce que** la substance active pharmaceutique est une substance active appartenant au groupe des antidiabétiques, des analgésiques, des anti-inflammatoires, des substances pour le traitement des affections rhumatismales, des anti-hypotenseurs, anti-hypertenseurs, des substances psycho-pharmacologiques, des tranquillisants, des antiémétiques, des myorelaxants, des glucocorticoïdes, des médicaments pour le traitement des colites ulcéreuses ou de la maladie de Crohn, des antihistaminiques, des antibiotiques, des anti-épileptiques, des anticoagulants, des antifongiques, antitussifs, médicaments pour le traitement de l'artériosclérose, des diurétiques, des enzymes, des inhibiteurs d'enzymes, des médicaments pour le traitement de la goutte, des hormones et leurs inhibiteurs, des glucosides cardiotoniques, des substances pour l'immunothérapie et des cytokines, des laxatifs, des normolipémiants, des médicaments anti-migraine, des préparations à base de substances minérales, des substances pour le traitement des affections otologiques, des antiparkinsoniens, des substances pour le traitement des affections de la thyroïde, des spasmolytiques, des inhibiteurs d'agrégation des thrombocytes, des vitamines, des cytostatiques et des inhibiteurs de métastases, des substances de phytopharmacologie, des substances de chimiothérapie et des acides aminés.

**9.** Composition selon l'une quelconque des revendications 1 à 8, **caractérisée en ce que** la substance active pharmaceutique est une substance active du groupe des analgésiques, des médicaments pour le traitement des colites ulcéreuses ou de la maladie de Crohn, des corticostéroïdes, des inhibiteurs de la pompe à protons, des substances pour l'état de mal viral, des normolipémiants, des bloquants H2, des antibiotiques et des inhibiteurs de l'enzyme de conversion de l'angiotensine.

**10.** Composition selon l'une quelconque des revendications 1 à 9, **caractérisée en ce que** la substance active pharmaceutique est le tramadol, la morphine, l'acide 5-aminosalicylique, le budésonide, l'oméprazole, l'acyclovir, la simvastatine, la pravastatine, la ranitidine, la famotidine, l'amoxicilline, l'acide clavulanique, l'énalapril, l'amlodipine ou un sel pharmaceutiquement acceptable ou un dérivé de celui-ci.

**11.** Composition selon l'une quelconque des revendications 1 à 10, sous forme de comprimés, dragées, gélules, comprimés pelliculés, comprimés en dispersion, comprimés sublinguaux, comprimés effervescents, sachets, poudres pour sirops ou suppositoires.

12. Composition selon l'une quelconque des revendications 1 à 11, sous forme de comprimés contenant de la cellulose microcristalline, de la polyvinylpyrrolidone soluble dans l'eau et de la polyvinylpyrrolidone à réticulation transversale insoluble dans l'eau, formant des substances auxiliaires pour former des comprimés.

13. Composition selon l'une quelconque des revendications 1 à 12, sous forme de comprimés à effet retardé sécables.

14. Procédé de préparation d'une composition pharmaceutique selon l'une quelconque des revendications 1 et 3 à 13, **caractérisé en ce que** la substance active pharmaceutique est mélangée à un polymère insoluble dans le suc gastrique et le suc intestinal et est compactée pour former une masse, de telle sorte que la masse compactée présente un diamètre de pores intérieur moyen de 35 $\mu$m au maximum, mesuré au moyen de la porosimétrie au mercure à une pression comprise entre 1 000 et 4 000 bar, **en ce que** la masse compactée est fractionnée en particules et les particules sont enrobées d'un polymère insoluble dans le suc gastrique et le suc intestinal, et **en ce que**, si nécessaire, les particules enrobées sont présentées sous une forme administrable appropriée.

15. Procédé de préparation d'une composition pharmaceutique selon l'une quelconque des revendications 2 à 13, **caractérisé en ce que** la substance active pharmaceutique est mélangée à un polymère insoluble dans le suc gastrique et le suc intestinal et est compactée pour former une masse, de telle sorte que la masse compactée comporte un pourcentage de porosité de 27 % au maximum, **en ce que** la masse compactée est fractionnée en particules et les particules sont enrobées d'un polymère insoluble dans le suc gastrique et le suc intestinal, et **en ce que**, si nécessaire, les particules enrobées sont présentées sous une forme administrable appropriée.

16. Procédé selon la revendication 14 ou 15, **caractérisé en ce que** pour mélanger la substance active pharmaceutique avec le polymère insoluble dans le suc gastrique et le suc intestinal, la substance active est humidifiée avec une dispersion ou solution aqueuse et/ou organique du polymère et le mélange est granulé et séché.

17. Procédé selon l'une quelconque des revendications 14 à 16, **caractérisé en ce que** le compactage est effectué sous une pression d'au moins 5 kN par cm de longueur à compacter.